(19) Europäisches Patentamt
European Patent Office
Office européen des brevets

(11) Publication number: **0 269 109 B1**

(12) **EUROPEAN PATENT SPECIFICATION**

(45) Date of publication of patent specification: **26.08.92**

(51) Int. Cl.$^5$: **C07C 69/743**, C07C 67/343

(21) Application number: **87117490.0**

(22) Date of filing: **26.11.87**

(54) **Process for the preparation of dialkyl 2-vinylcyclopropane-1,1-dicarboxylates.**

(30) Priority: **28.11.86 US 936001**
**28.11.86 US 936004**

(43) Date of publication of application:
**01.06.88 Bulletin 88/22**

(45) Publication of the grant of the patent:
**26.08.92 Bulletin 92/35**

(84) Designated Contracting States:
**BE CH DE FR GB LI NL**

(56) References cited:
**EP-A- 0 025 846**
**EP-A- 0 050 762**
**US-A- 4 321 406**

(73) Proprietor: **HENKEL CORPORATION**
**300 Brookside Avenue**
**Ambler, Pennsylvania 19002(US)**

(72) Inventor: **Clark, Clarence E., Jr.**
**1816 Greenbriar Place**
**Cincinnati Ohio(US)**
Inventor: **Fayter, Richard G., Jr.**
**2746 Saturn Drive**
**Fairfield Ohio(US)**

(74) Representative: **Patentanwälte Grünecker, Kinkeldey, Stockmair & Partner**
**Maximilianstrasse 58**
**W-8000 München 22(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

## Description

G. S. Skinner, et al. first reported the condensation of 1,4-dihalo-2-butene and diethyl malonate in J. Am. Chem. Soc., 72, 1648(1950). The condensation was conducted under anhydrous conditions by reacting the dihalide with the pre-formed disodio anion of the malonic ester in an attempt to synthesize spirocyclopentane-1,5-barbiturates. Kierstead, et al. (J. Chem. Soc., 1952, 3610-21 and J. Chem. Soc., 1953, 1799) reported the preparation of diethyl 2-vinylcyclopropane-1,1-dicarboxylate by the condensation of 1,4-dibromo-2-butene and ethyl sodiomalonate and observed that continual attack by malonate and anion on the 2-vinylcyclopropane derivative produced side products, one of which was 2-vinylbutane-1,1,4,4-tetracarboxylate. Kierstead, et al. also extended the general reaction to ethyl cyanoacetate and ethyl acetoacetate to obtain the corresponding 2-vinylcyclopropane derivatives. In an attempt to develop a new synthetic route for the preparation of the cyclopentane counterparts by deoxyribonucleosides, Murdock, et al. in J. Amer. Chem. Soc., 27, 2395 (1962) reported condensing cis-1,4-dichlorobutene-2 with sodiomalonic ester under anhydrous conditions as the first step in their reaction sequence.

With all of the above reactions, as well as in other reports dealing with the condensation of malonic esters with 1,4-dihalo-2-butenes, e.g. Birch, et al., J. Org. Chem. 23, 1390 (1958); Schmid, et al., J. Org. Chem. 32, 254 (1967); Stewart, et al., J. Org. Chem., 34, 8 (1969), the metal alkoxide and malonic ester were prereacted to first form the corresponding sodiomalonate anion, which was then very slowly added to the dihalobutene. This procedure was considered essential for the successful conduct of the reaction and to optimize the yield of the vinylcyclopropane dicarboxylate. The dihalo compound was not combined directly with the alcoholic caustic to avoid ether by-product formation since this is a well known and widely used procedure (Williamson synthesis) for the preparation of ethers. By adding the malonate anion to the dihalobutene and carefully controlling the rate of this addition, it was believed that linear diaddition products formed by either continued attack on the vinylcyclopropane product by malonate anion or reaction of both the halogens on a single molecule would be minimized. Strictly anhydrous conditions were employed throughout the entire reaction procedure, i.e. during the formation of the anion and the addition of the anion to the dihalobutene, since it is generally accepted that for malonate and acetoacetic ester condensations the presence of water is detrimental (Practical Organic Chemistry, A. I. Vogel, 3rd Ed., Longmans, Green and Co., Ltd., London (1967) pp. 481-486). Even as late as 1970 the classical procedure first developed by Skinner and coworkers was still being used as evidenced by the report of Den Besten, et al. (J. Chem. Eng. Data, 15, 453 (1970)) who prepared diethyl 2-vinylcyclopropane-1,1-dicarboxylate for subsequent thermal decomposition.

In view of the complex state of the reagents, the requirement to operate under strictly anhydrous conditions and the necessity for a sophisticated reaction vessel to carry out the detailed addition, it has heretofore not been practical to prepare vinylcyclopropane derivatives on a commercial scale via such condensation reactions.

U.S Patents Nos. 4,328,168 and 4,328,169 inter alia, describe improved processes for the preparation of vinylcyclopropane derivatives. These processes are adaptable to commercial operation and involve reacting, in a fluid state, an alkylating agent, e.g. 1,4-dichlorobutene-2, and an activated methylene compound, e.g. dimethyl malonate, in the presence, respectively, of a cyclic polyether compound or an alkylene oxide derivative and an alkali metal compound. A wide variety of cyclopropane derivatives are readily obtained by these processes.

Another process which has met with favor in overcoming the disadvantages of the earlier literature processes is the phase-transfer-catalyzed synthesis of vinylcyclopropane derivatives described in U.S. Patent No. 4,252,739, among others, and which involves reacting an alkylating agent, e.g. 1-4-dichlorobutene-2, and an activated methylene compound, e.g. a lower alkyl malonate in the presence of an onium compound, an alkali metal compound and water. While this process works well with certain lower alkyl esters of 2-vinylcyclopropane-1,1-dicarboxylate, for example the ethyl and higher esters, it produces lower yields of the order of < 10% when dimethyl 2-vinylcyclopropane-1, 1-dicarboxylate is sought to be obtained. It appears that the phase-transfer process when directed to the synthesis of the dimethyl ester produces low yields due to ester saponification which is apparently competitive with the condensation reaction in the presence of methyl esters but which saponification is insignificant with the ethyl and higher esters.

It would be highly desirable, therefore, if an improved process for the preparation of di lower alkyl 2-2-vinylcyclopropane-1,1-dicarboxylates by the reaction of 1,4-dihalobutenes and malonic esters were available which did not possess the drawbacks of the prior art processes. It would also be desirable if it were possible to eliminate the need for conducting the process in a stepwise manner, i.e., preforming the anion, and if the need for maintaining strictly anhydrous conditions could be eliminated and if the yield of the

desired product could be increased, the process would have even greater utility. These and other advantages are realized by the improved process of this invention.

The starting material for the condensation of 1,4-dihalobutene-2 is preferably 1,4-dichlorobutene-2 and as a commercial product this is usually composed of three isomeric dichlorobutenes, trans-1,4-dichlorobutene-2, cis-1,4- dichlorobutene-2 and 3,4-dichlorobutene-1. Trans-1,4- dichlorobutene-2 is the preferred starting material as the stereochemistry of the intermediate (I) is such that the desired dimethyl 2-vinyl-cyclopropane-1,1-dicarboxylate is the exclusive product. This is illustrated in Mechanism I below:

MECHANISM I

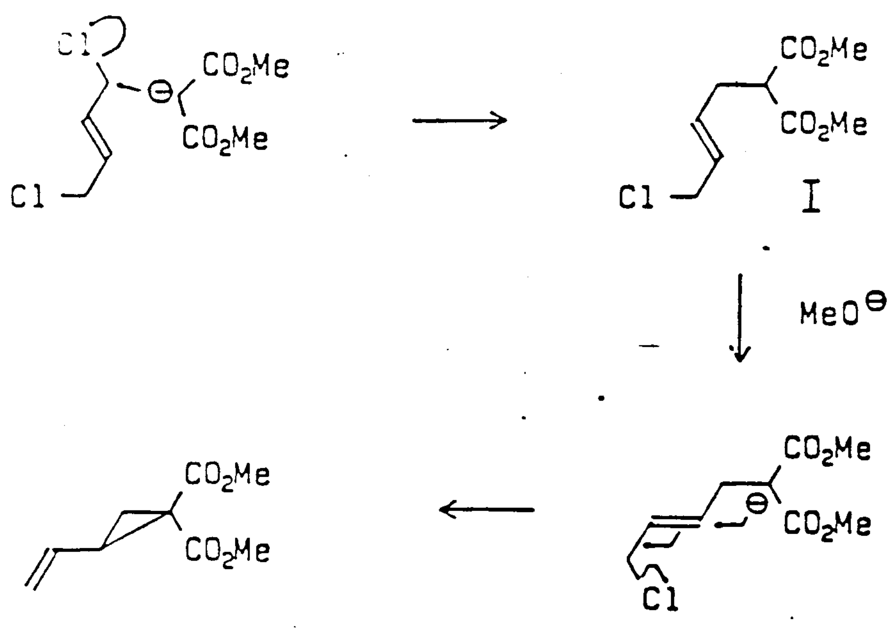


Cis-1,4-dichlorobutene-2 gives two products, dimethyl 2-vinylcyclopropane-1,1-dicarboxylate and dimethyl cyclopent-3-ene-1,1-dicarboxylate in nearly equal amounts as shown in Mechanism II below.

MECHANISM II

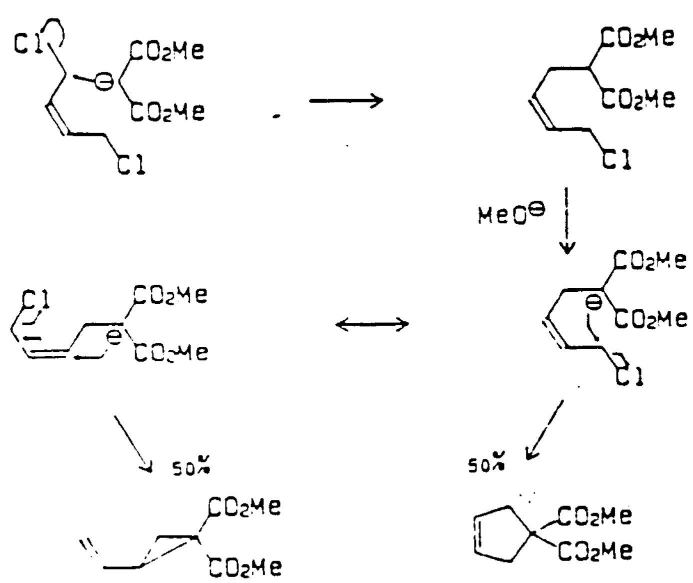

It is nearly impossible to separate dimethyl cyclopent-3-ene-1,1-dicarboxylate from the desired dimethyl 2-vinylcyclopropane-1,1-dicarboxylate by any reasonable means. In the described condensation reaction the third isomer, 3,4-dichlorobutene-1 gives only useless elimination products.

Fractional distillation of the three dichlorobutene isomers can readily be accomplished, but this is an expensive process and leads to the additional difficulty and cost of disposing of 3,4-dichlorobutene-1 and cis-1,4-dichlorobutene-2.

Efforts have been made in terms of isomerizing cis-1,4-dichlorobutene-2 to trans-1,4-dichlorobutene-2, but these methods have met with only moderate success.

Heterogeneous iron, tin and copper compounds as well as onium salts have been reported in the literature as dichlorobutene isomerization catalysts. A typical process employing a copper catalyst is disclosed in U.S. Patent No. 2,911,450. However, such processes are not completely satisfactory as they either have proven to be ineffective in some instances or have given equilibrium mixtures of all three dichlorobutenes.

The use of thiols as cis-to-trans-olefin isomerization catalysts has also been reported in the literature. See, W. G. Niehaus, Jr., Bioorg. Chem., 3(3), 302-10 (1974) and C. Walling, et al., J. Amer. Chem. Soc., 81, 1144-8 (1959) as has hydrogen bromide-catalyzed isomerization. See N. P. Neureiter, et al., J. Amer. Chem. Soc., 82, 5345-8 (1960). However, the thiol-catalyzed and hydrogen bromide-catalyzed isomerization of olefins typically leads to an equilibrium mixture of approximately 80% trans-and 20% cis-olefin. This appears to be true regardless of whether the starting olefin is cis or trans. See C. Walling, et al. Ibid.

It would be highly desirable, therefore, if an improved process could be developed which would permit an efficient cis-to-trans isomerization of 1,4-dichlorobutene-2 so that a high trans ( > 90%) mixture could be obtained from the usual commercial mixture of 1,4-dichlorobutene-2, which normally has a trans/cis ratio of 77/23, or from other mixtures having even lower trans content without any of the attendant disadvantages of the prior art.

It would also be highly desirable to provide a product with a high content of trans-1,4-dichlorobutene-2 and being substantially free from the other two isomers cis-1,4-dichlorobutene-2 and 3,4-dichlorobutene-1, which in the described condensation reaction with malonic esters either give approximately equal amounts of the desired dimethyl 2-vinylcyclopropane-1,1-dicarboxylate and the unwanted dimethyl cyclopent-3-ene-1,1-dicarboxylate or in the case of the isomeric 3,4-dichlorobutene-1 only useless elimination products.

The present invention relates to a process for preparing dialkyl 2-vinyl-cyclopropane-1,1-dicarboxylates which comprises condensing a di-lower alkyl malonic ester with a 1,4-dihalobutene-2 in the presence of an alcoholic solution of metallic alkoxide and recovering the dialkyl-2-vinylcyclopropane-1,1-dicarboxylate so produced. Furthermore, the 1,4-dihalobutene-2 is preferably in the trans form and is formed from the isomerization of 1,4-dihalobutenes-2 by contacting a mixture of trans-1,4-dihalobutene-2 and cis 1,4-dihalobutene-2 with an isomerization catalyst and an initiator at a temperature sufficient and time sufficient to permit the conversion of substantially all of the cis-1,4-dihalobutene-2 to trans-1,4-dihalobutene-2.

The present invention is based upon the discovery that high yields, of the order of 70% or higher, of di lower alkyl-2-vinylcyclopropane-1,1-dicarboxylates can be readily obtained by the addition of a alcoholic solution of a suitable metallic alkoxide to a solution of a malonic ester, and a 1,4-dihalobutene-2 in a lower alcohol solvent. The reaction is exothermic but is easily controlled by alcohol reflux. Workup consists of filtration, neutralization with a mineral acid, a second filtration, removal of solvent under vacuum and a final vacuum distillation. Crude yields are consistently 80-85% with distilled yields in the range of 75-80%.

The process of the present invention, therefore, relates to the preparation of di lower alkyl-2-vinylcyclopropane-1,1-dicarboxylates and more particularly is concerned with the preparation of such compounds by a novel process comprising the addition of an alcoholic metallic alkoxide to a solution of an olefin, for example 1,4-dihalobutene-2 and a malonic ester. The reaction is preferably carried out in the presence of an inert organic solvent, e.g. a lower alcohol, at reflux temperatures. Preferably the procedure involves the rapid addition of 25% methanolic sodium methoxide to a solution of 1,4-dichlorbutene-2 and dimethyl malonate in a minimum amount of methanol. The reaction temperature is maintained at 65-70 °C. by methanol reflux and maintenance of a low temperature by the slow addition of methoxide is not needed. After the reaction is complete, the mixture is vacuum filtered, neutralized preferably with concentrated hydrochloric acid and filtered a second time to complete the removal of all salts. The solvent is then removed under vacuum to produce a yield of crude product of 80-85%. Final vacuum distillation produces a product of 75-80% yield.

Suitable organic solvents for the reaction include the lower alcohols, for example, methanol, ethanol, propanol, and the like, methanol being preferred for ease of handling.

Suitable metallic alkoxides include, for example, sodium or potassium methoxide, ethoxide, propoxide, butoxide, and the like. Again, a methanolic sodium methoxide solution is preferred.

The reaction may be neutralized with any strong mineral acid, e.g. sulfuric acid, hydrochloric acid, etc.

It is preferred that the halo group in 1,4-dihalobutene be chloro or bromo.

Suitable halogenated olefins for use in the present invention include:

1,4-dichlorobutene-2; 1,4-dibromobutene-2; 1-bromo-4-chlorobutene-2; 1,4-dichloro-2-methylbutene-2; 1,4-dibromo-2-methylbutene-2; 1,4-dichloro-2,3-dimethylbutene-2; 1,4-dibromo-2,3-dimethylbutene-2; 1,4-dichlorobutene-2; 1,4-dibromobutene-2; 1,4-dichloro-2-methylbutene-2; and 1,4-dibromo-2-methylbutene-2;

1,4-Dichloro- and 1,4-dibromobutene-2 are particularly useful for the present process in view of their commercial availability, reactivity and ability to yield highly useful vinylcyclopropane derivatives with minimal undesirable by-product formation.

It is preferred that the trans 1,4-dilobutene-2 be used in the present process. Particularly preferred trans 1,4-dihalobutene-2 olefins for use in the present process are the trans-1,4-dichlorobutene-2 and trans-1,4-dibromobutene-2 useful for the present process in view of their commercial availability, reactivity and ability to yield highly useful vinyl cyclopropane derivatives with minimal undesireable by-product formation. The trans isomers one obtained by an isomerization process.

A mixture of cis- and trans-1,4-dichlorobutene-2 can be isomerized to a high level of trans-1,4-dichlorobutene-2 by the catalytic influence of an isomerization catalyst, such as thiols or anhydrous hydrogen bromide or hydrogen chloride with an initiator, such as ultraviolet light and/or chemical initiators so that an 80/20 trans/cis mixture is isomerized to a 95/5 trans/cis mixture in as little as ten minutes followed immediately by the cyclocondensation with dialkyl malonate and metallic alkoxide.

The temperature of the isomerization reaction is not critical and may conveniently be from room temperature up to 80°C. or higher depending upon the catalyst employed for the isomerization.

Likewise, the amount of catalyst is not critical and may conveniently be from 0.5 mole % based on the weight of the dichlorobutene to about 20 mole % and preferably from 5 mole % to 10 mole %.

The time of the reaction is likewise not critical and depends to some extent upon the catalyst employed for the isomerization. Thus with the thiol catalyzed isomerization the time may range from 30 minutes to an hour or more at reaction temperatures of from 70°C. to 90°C. whereas with the anhydrous hydrogen bromide or chloride catalyzed isomerization the time is frequently from twenty to thirty minutes or so at temperatures preferably at about room temperature.

With both the thiol catalyzed and hydrogen bromide and chloride catalyzed isomerizations, ratios better than 93/7 trans/cis- dichlorobutene-2 have consistently been obtained with 95-97% recovery of the dichlorobutene-2.

Typical thiols useful in the described isomerization reaction are 2-mecaptoethanol, thiophenol, thiolacetic acid, methanethiol, thioglycolic acid, mercaptosuccinic acid, etc.

In the thiol catalyzed isomerization of the dihalobutenes as well as in the anhydrous hydrogen bromide or chloride isomerization reaction it is necessary to employ an initiator for the reaction. Typical chemical initiators may be, for example, 2,2′-azobisisobutyronitrile (AIBN), benzoyl peroxide, t-butyl peroxide, etc.

The amount of chemical initiator employed in the reaction is not critical but must be present in sufficient amount to initiate the reaction. Typically from about 0.1 mole % to about 5 mole % based on the weight of the dichlorobutene has been found to be effective.

As indicated above, 2-mercaptoethanol as the catalyst and 2,2′-azobisisobutyronitrile (AIBN) as the initiator are preferred and have been found to be highly useful in the isomerization of dichlorobutene as they consistently provide ratios greater than 93/7 trans/cis-dichlorobutene with 95-97% recovery of the dichlorobutenes.

Hydrogen bromide with either AIBN or ultraviolet light has also been found to be effective in producing remarkably high trans/cis (95/5) ratios of dichlorobutene at room temperature.

Hydrogen bromide is the preferred catalyst in the described reaction and has been found to be equally effective with either AIBN or ultraviolet light initiation. However, 2- mercaptoethanol with ultraviolet light and hydrogen chloride with ultraviolet light showed marginal activity and hydrogen iodide and $I_2$ showed no catalytic activity with either AIBN or ultraviolet light.

Suitable malonic esters for use in the present process are the lower alkyl malonates, such as dimethyl malonate, diethyl malonate, dibutylmalonate, disopropyl malonate, ethyl(N,N-dimethyl-2-aminoethyl)-malonate, and di(N,N-dimethyl-2-aminoethyl)malonate and the like, dimethyl malonate being preferred because of its ready availability.

The invention will be described in greater detail in conjunction with the following specific examples in which the parts are by weight unless otherwise specified.

Example 1

Dimethyl 2-Vinylcyclopropane-1,1-dicarboxylate
(Comparative example - not part of the present invention)

Sodium methoxide (108.02 g, 2.0 moles) 25% in MeOH was added slowly (~2.25 hrs.) to dimethyl malonate (132.12 g, 1.0 mole) in a heated and stirred flask having a bottom opening; 200 mL additional MeOH was required to maintain fluidity of the slurry. The sodiomalonate was then added (~30 min.) through the bottom opening to 1,4-dichlorobutene-2 (125 g, 1.0 mole) in a second heated and stirred flask. The mixture was heated at reflux ~4.5 hours, cooled, and vacuum filtered. The clear filtrate was then concentrated under vacuum at which point additional salts precipitated. An attempted second filtration was unsuccessful due to the slimy cake, and the salts were finally removed by centrifuging to give 146 g of crude product. Vacuum distillation (60°/0.4 mm - 90°/0.55 mm) gave a small forecut, 87.6 g of product (47.6% yield), and 44.5 g of residue.

Example 2

Dimethyl 2-Vinylcyclopropane-1,1-dicarboxylate

Sodium methoxide (108.02 g, 2.0 moles) 25% in MeOH was added in 40 minutes to dimethyl malonate (132.12 g, 1.0 mole), 1,4-dichlorobutene-2 (150 g, 1.2 moles), and 50 mL MeOH and allowed to stir at ambient temperature overnight. The mixture was vacuum filtered, neutralized with concentrated HCl to 3.5 - 4.0 pH, and filtered a second time. Solvent was then removed under vacuum to give 194.8 g of light yellow crude product. Vacuum distillation (55°/0.25 mm - 72°/0.4 mm) with 0.064 g of hydroquinone added gave 142.5 g of product (77.4% yield) and 30.4 g of residue.

Example 3

Isomerization of Dichlorobutene with 2-Mercaptoethanol and 2,2′-Azobisisobutyronitrile

To 10 mL of 1,4-dichlorobutene-2 was added 0.5 mL of 2-mercaptoethanol (7.5 mole % based on dichlorobutene and 0.15 g of 2,2′-azobisisobutyronitrile (0.97 mole %). The reaction was then stirred at 80°C with the following results:

| | |
|---|---|
| 0 minutes | 80.5/19.2 trans/cis |
| 15 minutes | 88.9/ 8.5 trans/cis |
| 30 minutes | 91.0/ 6.7 trans/cis |

Example 4

Isomerization of Dichlorobutene with HBr and UV Light

Approximately 100 mL of 1,4-dichlorobutene-2 was saturated with anhydrous HBr by subsurface introduction through a fritted glass gas dispersion tube. HBr addition was terminated when persistent fumes were visible above the liquid surface. The mixture was then stirred at ambient temperature while being irradiated with a Pen-Ray*lamp with the following results:

| | |
|---|---|
| 0 minutes | 76.6/22.9 trans/cis |
| 5 minutes | 90.9/ 5.4 trans/cis |
| 10 minutes | 90.4/ 5.1 trans/cis |

Example 5

The procedure of Example 2 was followed except that trans-1,4-dichlorobutene-2 as obtained by the

* 2.5 watts output with 80-90% of radiation at 253.7 nm.

procedure of Example 3 was condensed with dimethyl malonate. High yields of dimethyl 2-vinylcyclopropane-1,1-dicarboxylate of high purity were obtained.

Example 6

The procedure of Example 2 was followed except that trans-1,4-dichlorobutene-2 as obtained by the procedure of Example 4 was condensed with dimethyl malonate. High yields of dimethyl 2-vinylcyclopropane-1,1-dicarboxylate of high purity were obtained.

Example 7

Combined HBr-catalyzed Isomerization and Cyclocondensation

~80% Trans-1,4-dichlorobutene-2 (150 g, 1.2 moles) and 2,2′-azobisisobutyronitrile (AIBN) (3.94 g, 0.024 mole) were heated with stirring to 60 °C at which point HBr gas addition was started. After 10 minutes, heating and gas flow were terminated (~94/6 trans/cis by GC) and the reaction allowed to cool with $N_2$ purge to remove HBr. Dimethylmalonate (132.12 g, 1.0 mole) in 50 mL of MeOH was then added to the flask. NaOMe (108.02 g, 2.0 moles) 25% in MeOH was added in 17 minutes and the reaction allowed to cool (80.5% dimethyl 2-vinylcyclopropane-1,1-dicarboxylate and 3.3% dimethyl cyclopent-3-ene-1,1-dicarboxylate by GC). After workup and distillation as above, 139.2 g (75.6% yield) of product consisting of 95.5% dimethyl 2-vinylcyclopropane-1,1-dicarboxylate and 4.5% dimethyl cyclopent-3-ene-1,1-dicarboxylate was obtained.

Example 8

Combined Thiol-catalyzed Isomerization and Cyclocondensation

To ~80% trans-, 20% cis-1,4-dichlorobutene-2 (150 g, 1.2 moles) was added 2-mercaptoethanol (7.03 g, 0.09 moles) and AIBN (1.9 g, 0.0116 moles). The mixture was then heated at 80 °C with stirring for 30 minutes at which point the trans/cis ratio was ~92/8. After cooling to 23 °C, and without further treatment, the cyclocondensation, workup, and distillation were conducted as in Example 2. Distillation afforded 114 g of product containing 95.5% dimethyl 2-vinylcyclopropane-1,1-dicarboxylate and 4.5% dimethyl cyclopent-3-ene-1,1-dicarboxylate.

## Claims

1. A process for preparing dialkyl 2-vinyl-cyclopropane-1,1-dicarboxylates which comprises condensing a di-lower alkyl malonic ester with a 1,4-dihalobutene-2 in the presence of an alcoholic solution of metallic alkoxide and recovering the dialkyl-2-vinylcyclopropane-1,1-dicarboxylate so produced.

2. The process according to Claim 1 in which the malonic ester is dimethyl malonate.

3. The process according to Claims 1 or 2 in which the 1,4-dihalobutene-2 is substantially trans 1,4-dihalobutene-2.

4. The process according to any of Claims 1-3 in which halo in 1,4-dihalobutene-2 is chloro or bromo.

5. The process according to any of Claims 1-2 and 4 in which the 1,4-dihalobutene is 1,4-dichlorobutene-2.

6. The process according to any of Claims 1-2 and 4 in which the 1,4-dihalobutene-2 is 1,4-dibromobutene-2.

7. The process according to any of Claims 1-5 in which the 1,4-dihalobutene is essentially trans 1,4-dichlorobutene.

8. The process according to any of Claims 1-4 and 6 in which the 1,4-dihalobutene is essentially trans 1,4-dibromobutene.

9. The process according to any of Claims 3-8 in which the trans 1,4-dihalobutene is formed from the isomerization of 1,4-dihalobutenes-2 by contacting a mixture of trans-1,4-dihalobutene-2 and cis 1,4-dihalobutene-2 with an isomerization catalyst and an initiator at a temperature sufficient and time sufficient to permit the conversion of substantially all of the cis-1,4-dihalobutene-2 to trans-1,4-dihalobutene-2.

10. The process according to Claim 9 in which the isomerization catalyst is a thiol catalyst.

11. The process according to any of Claims 9-10 in which the catalyst is 2-mercapto-ethanol.

12. The process according to Claim 9 in which the catalyst is anhydrous hydrogen bromide.

13. The process according to any of Claims 9-12 in which the initiator is 2,2′-azobisisobutyronitrile.

14. The process according to any of Claims 9 -12 in which the initiator is ultraviolet light.

15. The process according to any of Claims 1-14 in which the alcoholic solution is methanol, ethanol or propanol.

16. The process according to any of Claims 1-15 in which the metallic alkoxide is sodium methoxide, sodium ethoxide, sodium propoxide, sodium butoxide, potassium methoxide, potassium ethoxide, potassium propoxide and potassium butoxide.

17. The process according to any of Claims 1-16 in which the reaction temperatures range from about 25°C to about 80°C.

18. The process according to Claim 1 in which the dialkyl 2-vinylcyclopropane 1,1-dicarboxylate is dimethyl 2-vinylcyclopropane 1,1-dicarboxylate, the di-lower alkyl malonic ester is dimethyl malonate, the 1,4-dihalobutene-2 is 1,4-dichlorobutene-2, the alcoholic solution is 25% methanol and the metal alkoxide is sodium methoxide.

**Patentansprüche**

1. Verfahren zur Herstellung von Dialkyl-2-vinylcyclopropan-1,1-dicarboxylaten, wobei ein Di-nieder-alkylmalonsäureester in Gegenwart einer alkoholischen Lösung eines Metallalkoholats mit 1,4-Dihalogenbuten-2 kondensiert und das so hergestellte Dialkyl-2-vinylcyclopropan-1,1-dicarboxylat gewonnen wird.

2. Verfahren nach Anspruch 1, worin der Malonsäureester Dimethylmalonat ist.

3. Verfahren nach Anspruch 1 oder 2, worin das 1,4-Dihalogenbuten-2 im wesentlichen Trans-1,4-dihalogenbuten-2 ist.

4. Verfahren nach einem der Ansprüche 1 bis 3, worin das Halogen in 1,4-Dihalogenbuten-2 Chlor oder Brom ist.

5. Verfahren nach einem der Ansprüche 1 bis 2 und 4, worin das 1,4-Dihalogenbuten 1,4-Dichlorbuten-2 ist.

6. Verfahren nach einem der Ansprüche 1 bis 2 und 4, worin das 1,4-Dihalogenbuten-2 1,4-Dibrombuten-2 ist.

7. Verfahren nach einem der Ansprüche 1 bis 5, worin das 1,4-Dihalogenbuten im wesentlichen Trans-1,4-dichlorbuten ist.

8. Verfahren nach einem der Ansprüche 1 bis 4 und 6, worin das 1,4-Dihalogenbuten im wesentlichen Trans-1,4-dibrombuten ist.

9. Verfahren nach einem der Ansprüche 3 bis 8, worin das Trans-1,4-dihalogenbuten durch Isomerisation von 1,4-Dihalogenbutenen-2 hergestellt wird, wobei ein Gemisch aus Trans-1,4-Dihalogenbuten-2 und Cis-1,4-dihalogenbuten-2 mit einem Isomerisationskatalysator bei einer Temperatur und einem Zeitraum, die ausreichen, um die Umwandlung von im wesentlichen allem Cis-1,4-dihalogenbuten-2 und Trans-1,4-dihalogenbuten-2 zu gestatten, miteinander in Kontakt gebracht werden.

10. Verfahren nach Anspruch 9, worin der Isomerisationskatalysator ein Thiokatalysator ist.

11. Verfahren nach einem der Ansprüche 9 bis 10, worin der Katalysator 2-Mercaptoethanol ist.

12. Verfahren nach Anspruch 9, worin der Katalysator wasserfreier Bromwasserstoff ist.

13. Verfahren nach einem der Ansprüche 9 bis 12, worin der Initiator 2,2'-Azobisisobutyronitril ist.

14. Verfahren nach einem der Ansprüche 9 bis 12, worin der Initiator ultraviolettes Licht ist.

15. Verfahren nach einem der Ansprüche 1 bis 14, worin die alkoholische Lösung Methanol, Ethanol oder Propanol ist.

16. Verfahren nach einem der Ansprüche 1 bis 15, worin das Metallalkoholat Natriummethoxid, Natriumethoxid, Natriumpropoxid, Natriumbutoxid, Kaliummethoxid, Kaliumethoxid, Kaliumpropoxid und Kaliumbutoxid ist.

17. Verfahren nach einem der Ansprüche 1 bis 16, worin die Reaktionstemperaturen ungefähr 25° C bis ungefähr 80° C betragen.

18. Verfahren nach Anspruch 1, worin das Dialkyl-2-vinylcyclopropan-1,1-dicarboxylat Dimethyl-2-vinylcyclopropan-1,1-dicarboxylat, der niedrige Dialkylmalonsäureester Dimethylmalonat, das 1,4-Dihalogenbuten-2 1,4-Dichlorbuten-2, die alkoholische Lösung 25 %-iges Methanol und das Metallalkoholat Natriummethoxid ist.

**Revendications**

1. Procédé de fabrication de vinyl-2 cyclopropane-dicarboxylates-1,1 de dialkyle, qui comprend la condensation d'un malonate de di-alkyle inférieur avec un dihalo-1,4 butène 2, en présence d'une solution alcoolique d'alcoolate métallique, et la récupération du vinyl-2 cyclopropane-dicarboxylate-1,1 de dialkyle ainsi obtenu.

2. Procédé selon la revendication 1, dans lequel le malonate est le malonate de diméthyle.

3. Procédé selon l'une des revendications 1 et 2, dans lequel le dihalo-1,4 butène-2 est le dihalo-1,4 butène-2 sensiblement trans.

4. Procédé selon l'une quelconque des revendications 1 à 3, dans lequel halo dans dihalo-1,4 butène-2 représente chloro ou bromo.

5. Procédé selon l'une quelconque des revendications 1, 2 et 4, dans lequel le dihalo-1,4 butène est le dichloro-1,4 butène-2.

6. Procédé selon l'une quelconque des revendications 1, 2 et 4, dans lequel le dihalo-1,4 butène-2 est le dibromo-1,4 butène-2.

7. Procédé selon l'une quelconque des revendications 1 à 5, dans lequel le dihalo-1,4 butène est le dichloro-1,4 butène sensiblement trans.

8. Procédé selon l'une quelconque des revendications 1 à 4 et 6, dans lequel le dihalo -1,4 butène est le dibromo-1,4 butène sensiblement trans.

**9.** Procédé selon l'une quelconque des revendications 3 à 8, dans lequel le trans dihalo-1,4 butène est formé par l'isomérisation de dihalo-1,4 butènes-2 par mise en contact d'un mélange de trans-dihalo-1,4 butène-2 et de cis-dihalo-1,4 butène-2 avec un catalyseur d'isomérisation et un initiateur, à une température suffisante et pendant un laps de temps suffisant pour permettre la conversion de sensiblement tout le cis-dihalo-1,4 butène-2 en trans-dihalo-1,4 butène-2.

**10.** Procédé selon la revendication 9, dans lequel le catalyseur d'isomérisation est un catalyseur de type thiol.

**11.** Procédé selon l'une quelconque des revendications 9 et 10, dans lequel le catalyseur est le mercapto-2 éthanol.

**12.** Procédé selon la revendication 9, dans lequel le catalyseur est le bromure d'hydrogène anhydre.

**13.** Procédé selon l'une quelconque des revendications 9 à 12, dans lequel l'initiateur est le 2,2'-azobisisobutyronitrile.

**14.** Procédé selon l'une quelconque des revendications 9 à 12, dans lequel l'initiateur est la lumière ultraviolette.

**15.** Procédé selon l'une quelconque des revendications 1 à 14, dans lequel la solution alcoolique est le méthanol, l'éthanol ou le propanol.

**16.** Procédé selon l'une quelconque des revendications 1 à 15, dans lequel l'alcoolate métallique est le méthylate de sodium, l'éthylate de sodium, le propylate de sodium, le butylate de sodium, le méthylate de potassium, l'éthylate de potassium, le propylate de potassium et le butylate de potassium.

**17.** Procédé selon l'une quelconque des revendications 1 à 16, dans lequel les températures de réaction se situent dans la plage allant d'environ 25 ° C à environ 80 ° C.

**18.** Procédé selon la revendication 1, dans lequel le vinyl-2 cyclopropane-dicarboxylate-1,1 de dialkyle est le vinyl-2 cyclopropane-dicarboxylate-1,1 de diméthyle, le malonate de di-alkyle inférieur est le malonate de diméthyle, le dihalo-1,4 butène-2 est le dichloro-1,4 butène-2, la solution alcoolique est constituée par du méthanol à 25%, et l'alcoolate métallique est le méthylate de sodium.